# EUROPEAN PATENT SPECIFICATION

(11) **EP 2 499 477 B1**
(45) Date of publication and mention of the grant of the patent: **25.02.2015**
(21) Application number: 10790687.7
(22) Date of filing: 10.11.2010
(51) Int. Cl.: G01N 1/38, G01N 1/40

(54) **DEVICE FOR DETECTING VOLATILE SUBSTANCES, APPARATUS USING SAID DEVICE AND REVELANT OPERATIVE METHOD**
VORRICHTUNG FÜR DEN NACHWEIS VON FLÜCHTIGEN STOFFEN, GERÄT MIT DIESER VORRICHTUNG UND ZUGEHÖRIGES BETRIEBSVERFAHREN
DISPOSITIF POUR DÉTECTER DES SUBSTANCES VOLATILES, APPAREIL UTILISANT LEDIT DISPOSITIF ET PROCÉDÉ DE FONCTIONNEMENT ASSOCIÉ

(30) Priority: 11.11.2009 IT RM20090581
(43) Date of publication of application: 19.09.2012
(73) Proprietor: IRCCS Azienda Ospedaliera Universitaria San Martino-IST-Istituto Nazionale per la Ricerca sul Cancro, 16132 Genova (IT); Omega S.r.L., 16147 Genova (IT)
(72) Inventor: CHIARUGI Luca, 16147 Genova (IT); TONIN Gian Paolo, 16132 Genova (IT); RUZZON Tiziana, 16132 Genova (IT)
(74) Representative: Tiburzi, Andrea
(86) International application number: PCT/IT2010/000448
(87) International publication number: WO 2011/058592

(56) References cited:
- EP-A1- 1 471 343
- EP-A1- 1 584 912
- US-A- 4 541 268
- US-A- 5 741 984

## Description

The present invention relates to a device for detecting volatile substances, to an apparatus using said device and to the relevant operative method.

More specifically, the invention concerns a device that can sample and analyse substances that are present within liquid and/or solid samples in an amount in the order of some micro litres, detecting volatile portion. Said device can be easily employed and ensures high operation stability.

Specification will be in the following addressed to detection of volatile substances contained in urine, and particularly to detection of markers useful to diagnosis of neuroblastoma and to monitor course of disease. It is well evident that said device must not be considered limited to this specific use.

At present, for diagnosis of neuroblastoma it is necessary detecting clinical-biological parameters, among which determination of homovanillic and vanilmandelic acid within patient urine. Nowadays, examination of said acids within urines is carried out with specialised centres by HPLC (High Pressure Liquid Chromatography), i.e. an expensive and difficult analysis.

Examples of device for the measurement of volatile substances are known from patent applications EP 1584912, US 4541268 and EP 1417343.

The patent application US 5741984 describes an apparatus for the measurement of volatile substances according to the preamble of claim 1.

In view of the above, it is an object of the present invention that of suggesting a device that can carry out measures of volatile substances with a high stability, quickly and at very low costs.

Another object of the present invention is that of suggesting a device for detecting and measuring volatile substances, that can be easily used even by those not skilled in the field.

It is therefore specific object of the present invention a device for detecting volatile substances according to claim 1.

Still according to the invention, ratio between said port of said analysis opening and the sum of the ports of said one or more outlet openings is lower and/or equal than 0,01.

Furthermore, according to the invention, said containment box has a bottom base, where said inlet opening is made, and a top base, opposed with respect to said bottom base, where said one or more outlet openings and said analysis opening for the introduction of said probe are made, the number of said outlet openings being equal to the number of said one or more sensors,

Advantageously, according to the invention, said detection device comprises a body arranged within said containment box, said body being provided with a through channel that has a first end communicating with said analysis opening and a second end communicating with the internal of said containment box.

Always according to the invention, said containment box can be a removable body.

Still according to the invention, said one or more sensors can be installed on the internal lateral surface of said containment box.

Furthermore, according to the invention, said containment body has a cylindrical shape and said body has a substantially spheroidal shape, said body radially providing on its surface a plurality of channels, in a number corresponding to the number of said sensors, said channels ensuring a sufficient and even partialization of said gas, and thus of the sample, toward each one of said sensors.

Advantageously, according to the invention, said body can have one of the following features: it is made of a insulating material, so that when said probe is inserted within said channel, said sample of a substance to be examined does not evaporate while said probe is inserted through said channel; or it is made in such way that said through channel has a first portion made up of an insulating material, so that said sample of a substance to be examined does not evaporate while said probe is inserted in the part of the through channel of said first portion, and a second portion made up of a conducting material, so that, while said probe is inserted through the part of the through channel of said second portion, said sample of a substance to be examined is modified to said set beforehand internal temperature of said containment box.

Always according to the invention, said device can comprise comprises flow distribution means arranged in correspondence of said at least one inlet opening, said flow distribution means being suitable to distribute said gas flow through said one or more sensors, said flow distribution means comprising a porous separator, preferably made of glass, arranged so as to delimitate a diffusion chamber of said gas in correspondence of said inlet opening with respect to a measuring and vaporization chamber, wherein the volatile substances from said sample evaporate and are carried by said gas flow, by said channels of said central body, sample is sent toward said sensors.

Still according to the invention, said device can comprise means for controlling the internal temperature of said containment box, said control means preferably comprising a first thermoresistor installed within said diffusion chamber and a second thermoresistor installed within said measuring and vaporization chamber.

Advantageously, according to the invention, said substance to be examined is a liquid, preferably urine and/or serum and/or plasma and/or saliva and/or any other biological liquid.

It is further object of the present invention a detection apparatus characterized in that it comprises at least one detection and measure device as defined in the above; means for supplying said gas flow, comprising a gas cylinder, connected with said inlet opening by a conduct; means for adjusting the temperature of said gas out-flowing from said means for supplying said gas flow; at least one device for reducing the pressure of said gas; at least one flowmeter for adjusting said gas; and a control unit provided with a liquid crystal display and interaction members, said control unit being connected with said means for supplying said gas flow, with said sensors, with said first and second thermoresistor, with said means for adjusting the temperature, with said at least one device for reducing the pressure and with said flowmeter, said control unit being suitable to detect the temperature of said gas, to compare the same with the temperature of said diffusion chamber and of said measuring and vaporization chamber, so as to adjust said temperatures by said means for adjusting the temperature and said thermoresistors, to detect the measures of said sensors, and to show the outputs on said display.

It is also an object of the present invention a method for detecting volatile substances by means of said device for detecting volatile substances, comprising the following steps: providing a probe; treating, for example by immersion, the end of said probe in a sample of a substance to be examined, preferably a liquid sample, such as urine; and introducing said probe within said detection device as defined in the above through said analysis opening.

The present invention will be now described for illustrative, but not limitative purposes, according to its preferred embodiments, with particular reference to the enclosed drawings, wherein:
figure 1 shows a lateral section view of a device for detecting and measuring volatile substances according to the present invention;
figure 2 shows a plan section of a device for detecting and measuring volatile substances according to figure 1:
figure 3 shows a heating resistance for a device for detecting and measuring volatile substances according to the present invention;
figure 4 shows a second embodiment of detecting and measuring volatile substances according to the present invention;
figure 5 is a table wherein showing samples of substances subjected to testing and relevant amounts; and
figures 6 - 8 shows curves indicating, for example, responses of sensors to homovanillic and vanilmandelic acid obtained by the device according to the invention.

Similar parts will be indicated by the same references in the different views.

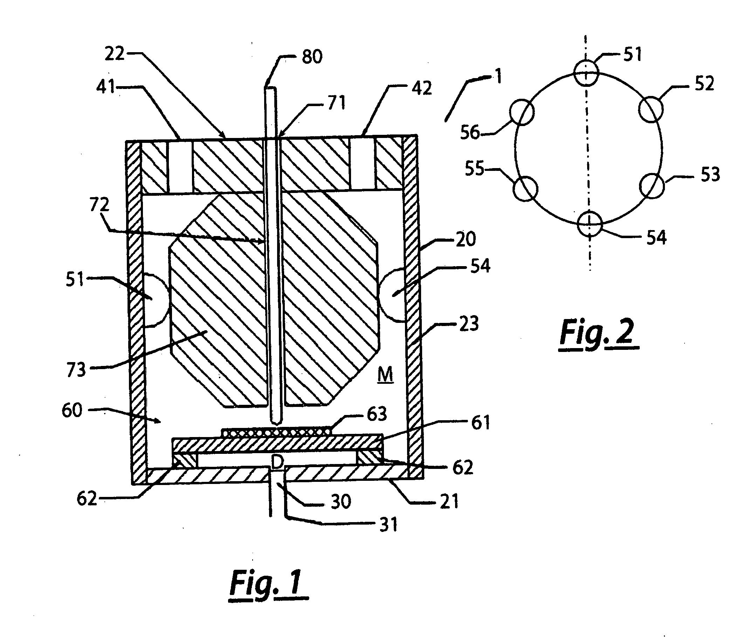

Making reference to figures 1 and 2, it is observed a device for detecting volatile substances according to the invention. Said device 1 comprises a containment box 20, within which there are a plurality of detection sensors that, in the present embodiment are six sensors and are indicated by reference numbers 51, 52, 53, 54, 55 and 56. A carrier gas is sent on said sensors 51 - 56, as better described in the following. Structure of containment box 20 realizes a diffusion chamber D and a vaporization and measurement chamber M. volatile parts of a sample of a substance to be subjected to analyses introduced beforehand and evaporated within containment box 20, are transported by flow of said carrier gas on said sensors 51 - 56.

Examining in greater detail device 1, it is observed that in the present embodiment, said containment box 20 has a cylindrical shape and a bottom base 21, an upper base 22 and a lateral wall 23.

An opening 30 is obtained on said bottom base 21, in order to introduce said carrier gas within said diffusion chamber D, under pressure by a conduct 31. Entering carrier gas flow it heated up before, so that it has the same inner temperature of said containment box 20.

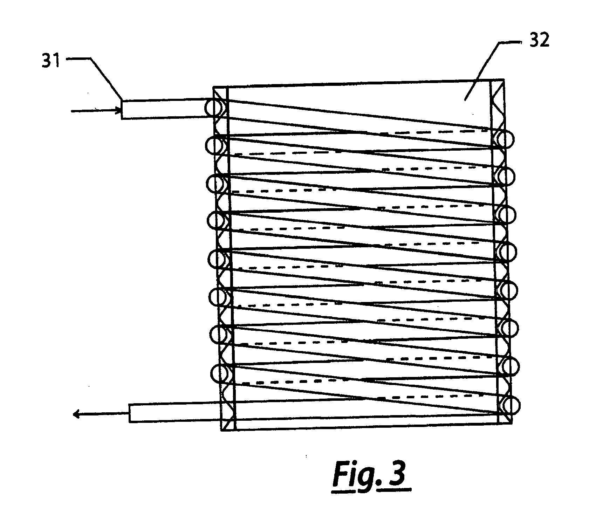

Figure 3 shows an embodiment of said heating means of the device 1, comprising a heating band-like resistance 32, on which conduct 31 is wound, like a coil.

Gas employed is preferably gas chromatographic grade gas, having the required purity in order to prevent that substances are introduced to which sensors 51 - 56 could be sensitive that would jeopardize the measurement results. Furthermore, gas flow is adjusted by a pressure reducer and monitored by a flowmeter.

Thus, carrier gas enters within containment box at a temperature avoiding every condensation that could occur in case gas carrier temperature is lower than temperature causing evaporation of volatile parts within sample introduced. In fact, possible condensation randomly varies concentration of evaporated substances within carrier gas, thus making unreliable analyses.

In view of high sensitivity of a measurement system suitable to analyze micro amounts, it is necessary a remarkable stability of carrier gas flow that, if under static conditions, does not create particular problems, but during introducing can cause limitations. Thus, device 1 provides a particular geometry.

Coming back to figures 1 and 2, it is observed that six outlet openings for carrier gas are obtained on said upper base 22, two of which are shown in the section view, respectively indicated by reference numbers 41 and 42. Said openings are circularly provided and at the same distance each other in the present embodiment.

Number of sensors 51 - 56 is the same number of said carrier gas outlet openings; moreover, the latter are provided in correspondence of sensors 51 - 56, so as to ease outlet of carrier gas flow from containment box 20. Said sensors 51 - 56 can be of different type and different number, or they can all the same.

Furthermore, among most versatile sensors, it is possible indicating conductivity variation sensors, piezoelectric sensors, quart microbalance (QCM), surface acoustic wave sensors (SAW), MOSFET sensors, thermo conductivity sensors.

Device 1 also provides gas diffusion means 60, defining said diffusion chamber D. said gas diffusion chamber 60 are suitable to diffuse gas entering through said opening 30 within said vaporization and measurement chamber M, to send it toward said sensors 51 - 56.

Particularly, said gas diffusion means 60 comprise a porous septum 61, preferably comprised of glass, provided with a plurality of through holes (not shown in the figures). Said porous septum 61 is spaced with respect to opening 30 by a ring 62. Said porous septum 61 and said ring 62 define diffusion chamber D. said porous septum 61 ensures a capillary diffusion of gas flow, preventing preferred ways, and through channels realized within the central body 73 it is ensured right partialization of flow toward every sensor. Said channels are vertical milling, with a depth of about 10 mm, realized on lateral wall of body 73. In case six sensors are provided, said channels have a 60° distance between axes. Distance between axis angle of 360° is divided by the number of sensors.

Furthermore, a first thermo resistance (not shown in the figure) is provided within said diffusion chamber D for controlling temperature within the containment box 20. Furthermore, a Teflon^{®} disk 63 is provided on said porous septum, the function of which will be better described in the following.

In order to make detection, it is necessary placing substance to be subjected to examination within the box, so that it can be taken from gas flow and sent toward sensors 51 - 56. To this end, an analysis opening 71 is obtained on the upper base 22 of the containment box 20, communicating with said vaporization and measurement chamber M by a through channel 72 of a body 73, preferably a removable body, provided within said containment box 20. Said body 73 has a substantially spherical shape. Dimensions and shape of said body 73 are particularly suitable to ensure a partialization of gas flow (transporting extracted substances) uniform on said sensors 51 - 56. Each sensor 51 - 56 is thus interested by the same volume of sample. By the above solutions it is possible obtaining specific response signals that are solely a function of the substance amount, without any interference due to the amount interesting every single sensor 51 - 56 employed. In other words, amount of extracted substances from sample interesting every single sensor is exactly the same amount for each one of them, thus preventing response signal depending on the amount and on the quality of the substance to be subjected to examination.

It must be taken into consideration that number of sensors can be varied. Generally speaking, no structural modification is necessary with a number of sensors between 1 and 12. Furthermore, said sensors 51 - 56 can be fixed to the inner surface of wall 23 of said containment box 20 or, preferably, to said body 73. In the latter case, being said body removable, replacing the same, partializing said carrier gas flow toward single sensors, it can be easily modified number of sensors 51 - 56. Obviously, type and number of sensors to be used only depends on substances to be subjected to examination and to quantify.

Said through channel 72 is substantially provided in correspondence of the symmetry axis. Said body 73 of the present embodiment is comprised of insulating material, in order to prevent that, while substance to be subjected to examination is introduced through channel 72 within measurement and vaporization chamber M, said substance to be subjected to examination evaporate before arriving at the same chamber.

To position the sample of the substance to be subjected to examination, it is provided the use of a sampling probe 80, that can be realised as a rod, a closed capillary or like, in any case having an elongated shape, and comprised of a chemically inert material, resisting to temperature and having such a section to be able to pass through said analyses opening 71 and said channel 72.

Said sampling probe 80 is at least partially submerged (typically about 5 mm) within substance to be subjected to analysis, to be "wet" by the sample to be subjected to examination. The same result can be obtained by rubbing or by any other method permitting "wetting" a portion of said sampling probe 80. Sampling probe 80 so treated is introduced within the diffusion chamber D, within which, under the effect of temperature, volatile portion of the sample quickly passes to gaseous state and carrier gas flow sends it toward sensors 51 - 56.

When said sampling probe 80 is inserted within said channel 72 through said analysis opening 71, "wet" end reaches said porous septum 61. Teflon^{®} disk 63 is suitable to prevent that sampling probe 80 hits porous septum 61.

Carrier gas, through said diffusion chamber D, reaches glass porous septum 61 and is diffused within measurement cell M. Volatile substances contained within "wet" portion (end) of the probe, evaporate and gas carries them toward sensors 51 - 56. Then, gas flow exits from vaporization and measurement chamber M through said outlet openings.

Gas flow, through porous septum 61, thanks to the shape of said body 73, creates suitable flow paths within said body 73, within vaporization and measurement chamber M, and it is radially diffused toward every sensor 51 - 56. Six outlet openings and Teflon^{®} disk 63, centrally provided on said porous septum 61, create preferred paths through which gas, and thus volatile substances, flow.

Flow resistance due to said analysis opening 71, through which it is inserted or withdrawn the sampling probe 80, has a much higher value of flow resistance caused by said outlet openings 41 or 42 of said gas flow, so that introduction, or withdrawal, of sampling probe 80 from said through channel 72 does not create appreciable variations of carrier gas flow balance toward sensors 51 - 56. In a preferred embodiment, ratio between port of said analysis opening 71 and sum of ports of said outlet openings is in the order to 1/100 (0.01).

Said solution ensures flow stability necessary to repetitivity of responses from employed sensors that unavoidably would vary physical conditions (temperature, pressure and flow) of gas passing through them.

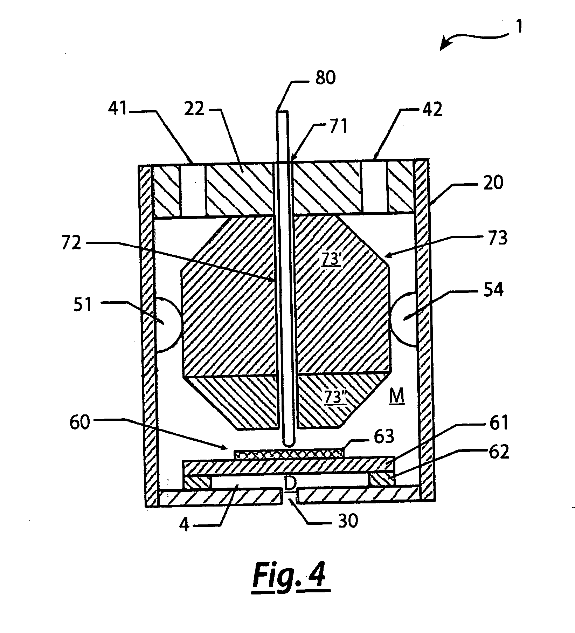

Figure 4 shows a further embodiment of device according to the invention, wherein body 73 is so realised that a first portion 73' is comprised of insulating material, so that channel 72 of said portion has walls comprised of said insulating material; and a second portion 73" is comprised of thermally conductive material, as walls of the relevant portion of channel 72.

Thus, when probe 80 with sample to be subjected to analysis is introduced within containment box 20, first insulated portion prevents that said sample can evaporate, while second final portion brings sample to temperature of said vaporization and measurement chamber M.

Preferably, a second PT100 thermo resistance (not shown in the figures) is installed in said vaporization and measurement chamber M, to detect temperature in order to permit a control and comparison with temperature of diffusion chamber D.

Usually, used sampling probe 80 can have different shape and dimensions, on the basis of the sample to be subjected to analysis. Anyway, maximum diameter must be 4 mm. Moreover, probes for solid samples and probes for liquid samples can be individuated.

Liquid sample probes are realised by a support rod, comprised of material resisting to temperature and chemically inert (glass, ceramics, stainless steel, ecc.). Said probes provide an end curve, which is "wet" by immersion within liquid, in order to take the sample to be subjected to analysis.

Solid sample probes are comprised of chemically inert materials, resisting to temperature and mechanically not deformable. Usually, they have a cylindrical shape, with a point tip, easing introduction within solid samples. Their use provides an introduction of the sample for the following withdrawal. Volatile parts, if present within sample, remain on probe, which are injected within device 1, introducing the probe within a suitable opening.

Solid sample probes can also be used for sampling volatile substances present in a gaseous atmosphere, such as, e.g. in human breath. In order to realise the above, it is necessary such an equipment which, by a Peltier battery system, refrigerates probe up to the temperature of -8°C (adjustable value). Refrigerated probe, if brought in contact with a gaseous atmosphere, "catches" volatile substances, freeing them and, when introduced within device 1, releases the same within gas, by which they are sent to sensors 51, 52, 53, 54, 55 and 56.

Device 1 can even operate in a suitable apparatus (not shown in the figures), essentially providing a gas flow generation unit comprising a cylinder connected with said conduct 31.

Said apparatus also comprises a control unit provided with a control panel with a liquid crystal display and interaction members such as a keyboard.

Said control unit is connected to said sensors 51 - 56, with said thermo resistances installed within said diffusion chamber D and within said vaporization and measurement chamber M, to pressure reducer and to flowmeter. By this solution, diffusion chamber D and vaporization and measurement chamber M are at the same temperature. This permits preventing that hottest or coldest spots along the evaporated substances path toward sensors 51 - 56 make concentration differences within gas, with consequent unreliable responses. In fact, in case points with different temperature are present within device 1, they would not permit a proper partialization of sample toward sensors 51 - 56 and, taking into consideration micro amounts introduced and vaporised, responses would not be proportional to gas concentration, but rather would randomly vary.

Temperature can be set at a maximum value set by sensor 51 - 56 maximum exercise temperature. Anyway, it is advisable choosing the highest possible temperature, to be sure that all volatile parts of the sample pass to the gaseous phase.

Adjustment of temperature is of the proportional type, permitting reaching a precision of 0.1 °C order.

Said adjustment stability is essential for sensor 51 - 56 response, both in a static phase and during analysis. Actual value can be continuously monitored by said display on said apparatus control panel.

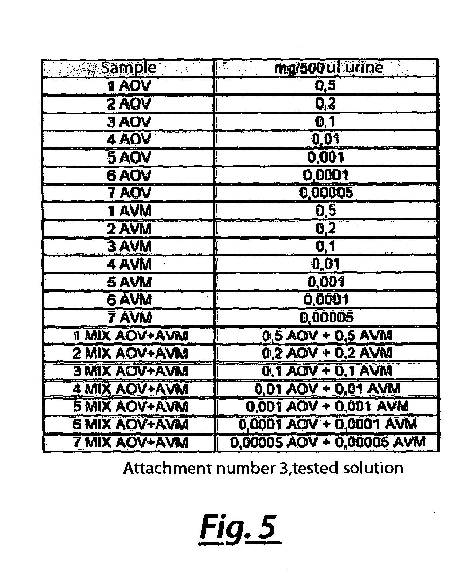

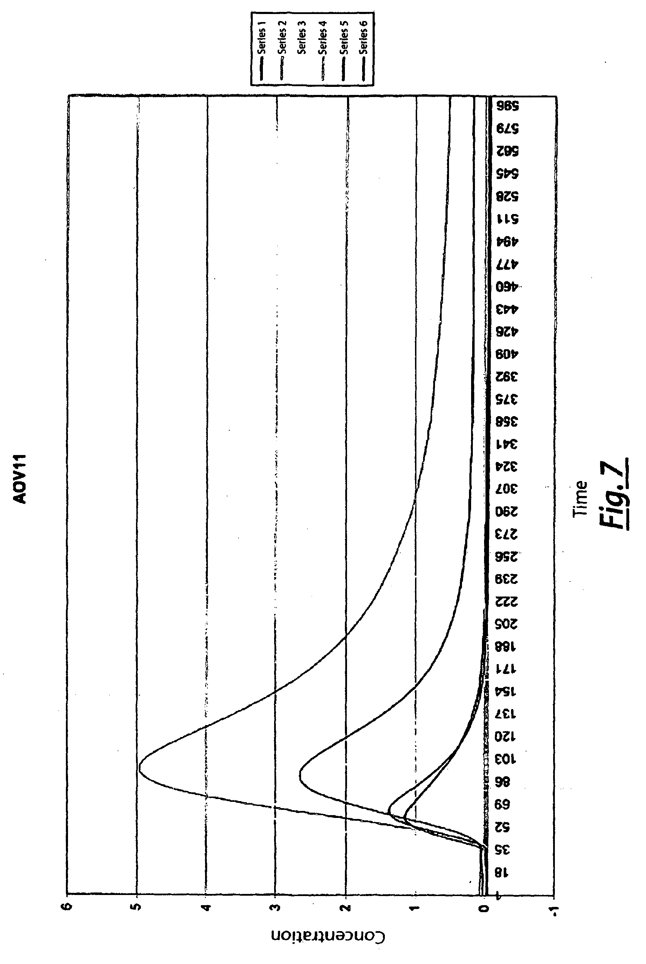

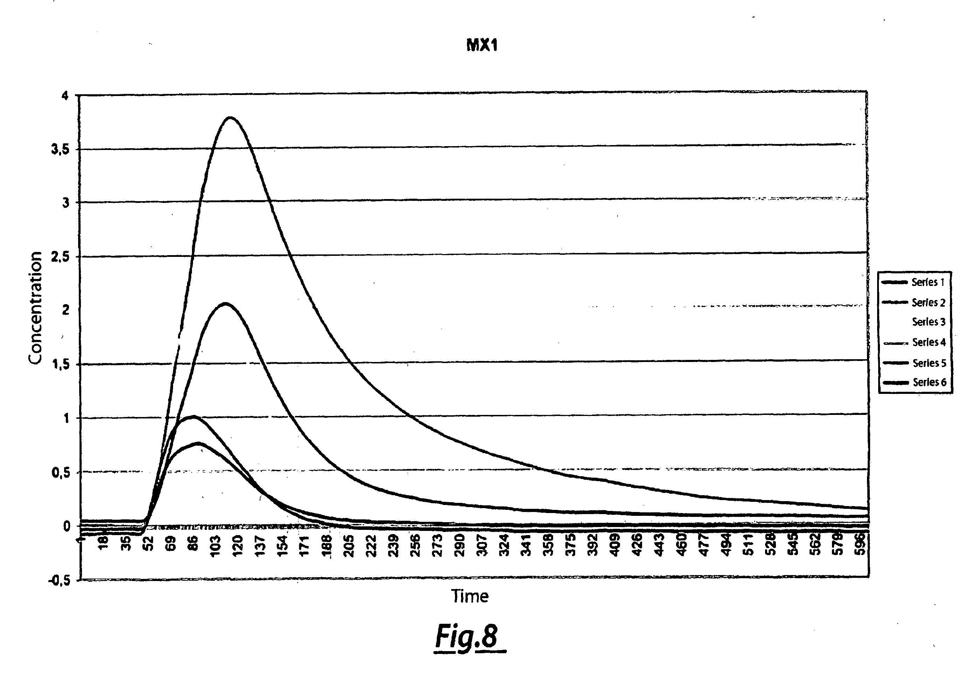

Figures 5 - 8 show some experimental data, with homovanillic acid and vanilmandelic acid as samples to be subjected to analysis, at different urine concentrations of healthy persons. Sampling and injection of the sample have been carried out as described in the above, i.e. wetting a probe end 80 and inserting the same within through channel 72.

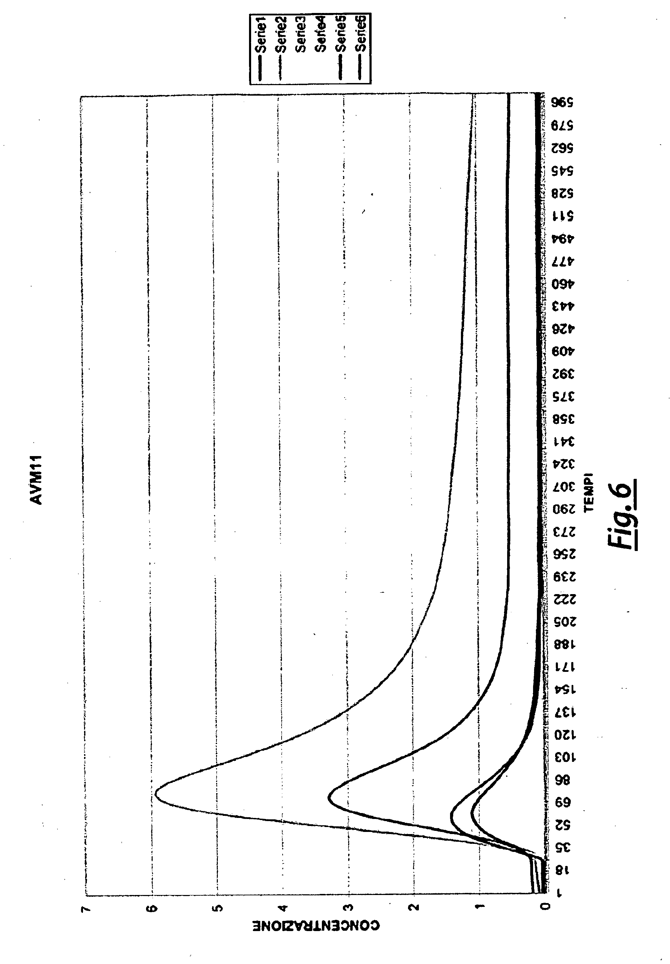

Figure 5 shows table of the solutions subjected to test, while figures 6 - 8 show graphs of responses of homovanillic acid and vanilmandelic acid while time passes, obtained by device according to the invention. Time is shown in abscissa and response to concentration of components of analyses substance is shown in ordinate. Lack of response from sensor 56 is noted from above graph. This is due to minimum, almost null, response from employed sensor for substances under examination. An advantage of the present invention is that detection device can detect small amounts of samples (order of micro litres) and it permits a very quick measurement. Furthermore, it is easy to use at low costs.

A further advantage of the invention is that device can be used in all apparatuses designed to analyse micro amounts of volatile substances in liquid but also solid samples, and thus it can be applied to biomedical, food, chemical, environmental, ecc fields.

The present invention has been described for illustrative, but not limitative purposes, according to its preferred embodiments, but it is to be understood that variations and/or modifications can be introduced by those skilled in the art without departing from the relevant scope, as defined in the enclosed claims.

## Claims

1. Device for the measurement of volatile substances comprising
a containment box (20), having at least one inlet opening (30) for the introduction of a gas flow, preferably chromatographic degree air, one or more outlet openings (41, 42) of said gas flow and at least one analysis opening (71),
a plurality of detection sensors (51, 52, 53, 54, 55, 56) arranged within said containment box (20), and
at least one probe (80), insertable within said containment box (20) through said at least one analysis opening (71),
**characterized**
**in that** the port of said analysis opening (71) is smaller than the sum of the ports of said one or more outlet openings (41, 42), so that the insertion or
the extraction of said probe (80) from said analysis opening (71) does not change the flow of said gas that reaches said sensors (51, 52, 53, 54, 55, 56),
**in that** said device comprises a body (73) arranged within said containment box (20), said body (73) being provided with a through channel (72) that has a first end communicating with said analysis opening (71) and a second end communicating with the internal of said containment box (20), and
**in that** said body (73) radially provides on its surface a plurality of channels, in a number corresponding to the number of said sensors (51, 52, 53, 54, 55, 56), said channels being realized on a lateral wall of body (73) and being V suitable for partialization of gas flow toward every sensor, so that, when said probe (80) is treated beforehand by a sample of a substance to be examined and it is inserted within said containment box (20), said gas flow uniformly carries the volatile substances from said sample to said plurality of sensors (51, 52, 53, 54, 55, 56)

2. Device (1) according to claim 1, **characterized in that** the ratio between said port of said analysis opening (71) and the sum of the ports of said one or more outlet openings (41, 42) is lower and/or equal than 0,01.

3. Device (1) according to anyone of the preceding claims, **characterized in that**
said containment box (20) has a bottom base (21), where said inlet opening (30) is made, and a top base (22), opposed with respect to said bottom base (21), where said one or more outlet openings (41, 42) and said analysis opening (71) for the introduction of said probe (80) are made, the number of said outlet openings (41, 42) being equal to the number of said one or more sensors (51, 52, 53, 54, 55, 56),

4. Device (1) according to anyone of the preceding claims, **characterized in that** said containment box (20) is a removable body.

5. Device (1) according to anyone of the preceding claims, **characterized in that** that said one or more sensors (51, 52, 53, 54, 55, 56) are installed on the internal lateral surface of said containment box (20).

6. Device (1) according to anyone of the preceding claims, **characterized in that** said containment box (20) has a cylindrical shape and said body has a substantially spheroidal shape.

7. Device (1) according to anyone of the preceding claims, **characterized in that** said body (73) has one of the following features:
- it is made of a insulating material, so that said sample of a substance to be examined does not evaporate while said probe (80) is inserted through said channel (72); or
- it is made in such way that said through channel (72) has a first portion (73') made of a insulating material, so that said sample of a substance to be examined does not evaporate while said probe (80) is inserted in the part of the through channel (72) of said first portion (73'), and a second portion (73") made of conducting material, so that, while said probe (80) is inserted through the part of the through channel (72) of said second portion (73"), said sample of a substance to be examined is modified to said set beforehand internal temperature of said containment box (20).

8. Device (1) according to anyone of the preceding claims, **characterized in that** it comprises flow distribution means (61, 62) arranged in correspondence of said at least one inlet opening (30), said flow distribution means (61, 62) being suitable to distribute said gas flow through said one or more sensors (51, 52, 53, 54, 55, 56),
said flow distribution means comprising a porous separator (61), preferably made of glass, arranged so as to delimitate a diffusion chamber (D) of said gas in correspondence of said inlet opening (30) with respect to a measuring and vaporization chamber (M), wherein the volatile substances from said sample evaporate and are carried by said gas flow, by said channels of said central body, sample is sent toward said sensors (51, 52, 53, 54, 55, 56).

9. Device (1) according to claim 8, **characterized in that** it comprises means for controlling the internal temperature of said containment box (20), said control means preferably comprising a first thermoresistor installed within said diffusion chamber (D) and a second thermoresistor installed within said measuring and vaporization chamber (M).

10. Device (1) according to anyone of the preceding claims, **characterized in that** said substance to be examined is a liquid, both as such and as vapor.

11. Device (1) according to anyone of the preceding claims, **characterized in that** said substance to be examined is a solid.

12. Device (1) according to anyone of the preceding claims, **characterized in that** it provides with a Peltier battery system for cooling said at least one probe (80) before sampling a volatile substance and inserting the same through said at least one analysis opening (71).

13. Detection and measurement apparatus **characterized in that** it comprises:
at least one detection and measure device (1) as defined in anyone of claims 1 - 12;
means for supplying said gas flow, comprising a gas cylinder, connected with said inlet opening (30) by a conduct (31);
means for adjusting the temperature of said gas out-flowing from said means for supplying said gas flow;
at least one device for reducing the pressure of said gas;
at least one flowmeter for adjusting said gas; and
a control unit provided with a liquid crystal display and interaction members, said control unit being connected with said means for supplying said gas flow, with said sensors (51, 52, 53, 54, 55, 56), with said first and second thermoresistor, with said means for adjusting the temperature, with said at least one device for reducing the pressure and with said flowmeter, said control unit being suitable to detect the temperature of said gas, to compare the same with the temperature of said diffusion chamber (D) and of said measuring and vaporization chamber (M), so as to adjust said temperatures by said means for adjusting the temperature and said thermoresistors, to detect the measures of said sensors (51-56), and to show the outputs on said display.

14. Method for detecting volatile substances by means of said device (1) for the measurement of volatile substances as defined in anyone of claims 1 - 12, comprising the following steps:
- providing a probe (80);
- treating, for example by immersion, the end of said probe (80) in a sample of a substance to be examined, preferably a liquid sample, such as urine; and
- introducing said probe (80) within said detection device (1) as defined in anyone of the preceding claims 1 - 12 through said analysis opening (71).

## Patentansprüche

1. Vorrichtung zur Messung von flüchtigen Substanzen, umfassend
einen Aufnahmebehälter (20), aufweisend zumindest eine Einlassöffnung (30) zur Einführung eines Gasflusses, vorzugsweise Luft chromatographischen Grades, eine oder mehrere Auslassöffnungen (41, 52) des Gasflusses und zumindest eine Analyseöffnung (71),
Detektionssensoren (51, 52, 53, 54, 55, 56), die innerhalb des Aufnahmebehälters (20) angeordnet sind, und
zumindest eine Sonde (80), die durch die zumindest eine Analyseöffnung (71) in den Aufnahmebehälter (20) einführbar ist,
**dadurch gekennzeichnet,**
**dass** der Eingang der Analyseöffnung (71) kleiner ist als die Summe der Eingänge der einen oder mehreren Auslassöffnungen (41, 42), so dass das Einfügen der Sonde (80) in oder das Herausführen der Sonde (80) aus der Analyseöffnung (71) nicht den Fluss des Gases beeinflusst, das die Sensoren (51, 52, 53, 54, 55, 56) erreicht,
**dass** die Vorrichtung einen innerhalb des Aufnahmebehälters (20) angeordneten Körper (73) umfasst, wobei der Körper (73) mit einem Durchgangskanal (72) versehen ist, der ein erstes Ende, das mit der Analyseöffnung (71) kommuniziert, und ein zweites Ende, das mit dem Inneren des Aufnahmebehälters (20) kommuniziert, aufweist, und
**dass** der Körper (73) radial auf seiner Oberfläche eine Vielzahl von Kanälen aufweist, in einer Anzahl, die der Anzahl der Sensoren (51, 52, 53, 54, 55, 56) entspricht, wobei die Kanäle auf einer Seitenwand des Körpers (73) vorgesehen sind und geeignet ausgeführt sind, um eine Aufteilung des Gasflusses hin zu jedem Sensor zu bewirken, so dass, wenn die Sonde (80) vorher mit einer Probe einer zu untersuchenden Substanz behandelt wird und in den Aufnahmebehälter (20) eingeführt wird, der Gasfluss die flüchtige Substanz gleichmäßig von der Probe zu der Vielzahl der Sensoren (51, 52, 53, 54, 55, 56) trägt.

2. Vorrichtung (1) nach Anspruch 1, **dadurch gekennzeichnet, dass** das Verhältnis zwischen dem Eingang der Analyseöffnung (71) und der Summe der Eingänge der einen oder mehreren Auslassöffnungen (41, 42) kleiner und/oder gleich 0,01 ist.

3. Vorrichtung (1) nach einem der vorhergehenden Ansprüche, **dadurch gekennzeichnet, dass** der Aufnahmebehälter (20) einen unteren Boden (21) aufweist, wo die Einlassöffnung (30) gefertigt ist, und einen in Bezug auf den unteren Boden (21) gegenüberliegenden oberen Boden (22) aufweist, wo die einen oder mehreren Auslassöffnungen (41, 42) und die Analyseöffnung (71) zur Einführung der Sonde (80) gefertigt sind, wobei die Anzahl der Auslassöffnungen (41, 42) gleich der Anzahl der einen oder mehreren Sensoren (51, 52, 53, 54, 55, 56) ist.

4. Vorrichtung (1) nach einem der vorhergehenden Ansprüche, **dadurch gekennzeichnet, dass** der Aufnahmebehälter (20) ein entnehmbarer Körper ist.

5. Vorrichtung (1) nach einem der vorhergehenden Ansprüche, **dadurch gekennzeichnet, dass** die einen oder mehreren Sensoren (51, 52, 53, 54, 55, 56) auf der inneren Seitenfläche des Aufnahmebehälters (20) installiert sind.

6. Vorrichtung (1) nach einem der vorhergehenden Ansprüche, **dadurch gekennzeichnet, dass** der Aufnahmebehälter (20) eine zylindrische Form hat, und dass der Körper eine im Wesentlichen kugelförmige Form aufweist.

7. Vorrichtung (1) nach einem der vorhergehenden Ansprüche, **dadurch gekennzeichnet, dass** der Körper (73) eines der folgenden Merkmale hat:
- er ist aus einem isolierenden Material gefertigt, so dass die Probe der zu untersuchenden Substanz nicht verdampft, während die Probe (80) durch den Kanal (72) eingeführt wird; oder
- er ist so hergestellt, dass der Durchgangskanal (72) einen ersten Bereich (73') aufweist, der aus einem isolierenden Material hergestellt ist, so dass die Probe der zu untersuchenden Substanz nicht verdampft, während die Probe (80) in den Teil des Durchgangskanals (72) des ersten Bereichs (73') eingeführt wird, und einen zweiten Bereich (73'') aufweist, der aus einem leitenden Material hergestellt ist, so dass, während die Probe (80) durch den Teil des Durchgangskanal (72) des zweiten Bereichs (73'') eingeführt wird, die Probe einer zu untersuchenden Substanz zu einer vorher festgelegten internen Temperatur des Aufnahmebehälters (20) verändert wird.

8. Vorrichtung (1) nach einem der vorhergehenden Ansprüche, **dadurch gekennzeichnet, dass** sie Strömungsverteilungsmittel (61, 62) umfasst, die korrespondierend zu der zumindest einen Einlassöffnung (30) angeordnet sind, wobei die Strömungsverteilungsmittel (61, 62) geeignet ausgeführt sind, um den Gasfluss über die einen oder mehreren Sensoren (51, 52, 53, 54, 55, 56) zu verteilen,
wobei die Strömungsverteilungsmittel einen porösen Teiler (61) umfassen, vorzugsweise aus Glas hergestellt, der so angeordnet ist, dass er eine Diffusionskammer (D) des Gases korrespondierend zu der Einlassöffnung (30) in Bezug auf eine Mess- und Verdampfungskammer (M) abgrenzt, wobei die flüchtigen Substanzen von der Probe verdampfen und von dem Gasfluss weggetragen werden, wobei die Probe von den Kanälen des zentralen Körpers den Sensoren (51, 52, 53, 54, 55, 56) zugeführt wird.

9. Vorrichtung (1) nach Anspruch 8, **dadurch gekennzeichnet, dass** sie Mittel zur Steuerung der internen Temperatur des Aufnahmebehälters (20) umfasst, wobei die Steuermittel vorzugsweise einen ersten Thermowiderstand umfassen, der innerhalb der Diffusionskammer (D) installiert ist, und einen zweiten Thermowiderstand umfassen, der in der Mess- und Verdampfungskammer (M) installiert ist.

10. Vorrichtung (1) nach einem der vorhergehenden Ansprüche, **dadurch gekennzeichnet, dass** die zu untersuchende Substanz eine Flüssigkeit ist, sowohl als solche und auch als Dampf.

11. Vorrichtung (1) nach einem der vorhergehenden Ansprüche, **dadurch gekennzeichnet, dass** die zu untersuchende Substanz ein Feststoff ist.

12. Vorrichtung (1) nach einem der vorhergehenden Ansprüche, **dadurch gekennzeichnet, dass** sie ein Peltier-Batteriesystem zur Kühlung der zumindest einen Sonde (80) aufweist, bevor eine flüchtige Substanz der Sonde ausgesetzt ist und vor dem Einführen der Sonde durch die zumindest eine Analyseöffnung (71).

13. Detektions- und Messgerät, **dadurch gekennzeichnet, dass** es umfasst:
eine Detektions- und Messvorrichtung (1), wie in einem der Ansprüche 1 bis 12 festgelegt;
Mittel zur Zuführung des Gasflusses, umfassend einen Gaszylinder, der mit der Einlassöffnung (3) über eine Leitung (31) verbunden ist;
Mittel zur Anpassung der Temperatur des Gases, das von dem Mittel zur Zuführung des Gasflusses herausfließt;
zumindest eine Vorrichtung zur Reduzierung des Drucks des Gases;
zumindest einen Durchflussmesser zur Anpassung des Gases; und
eine Steuereinheit, die mit einer Flüssigkristallanzeige und Interaktionselementen ausgestattet ist, wobei die Steuereinheit mit den Mitteln zur Zuführung des Gasflusses, mit den Sensoren (51, 52, 53, 54, 55, 56), mit dem ersten und zweiten Thermoelement, mit den Mitteln zur Anpassung der Temperatur, mit der zumindest einen Vorrichtung zur Reduzierung des Drucks und mit dem Durchflussmesser verbunden ist, wobei die Steuereinheit geeignet ausgeführt ist, um die Temperatur des Gases zu erfassen, die gemessene Temperatur mit der Temperatur der Diffusionskammer (D) und der Mess- und Verdampfungskammer (M) zu vergleichen, um die Temperatur durch die Mittel zur Anpassung der Temperatur und durch die Thermowiderstände anzupassen, um die Messsignale der Sensoren (51, 52, 53, 54, 55, 56) zu erfassen und um die Ausgaben auf der Anzeige anzuzeigen.

14. Verfahren zur Erfassung flüchtiger Substanzen mittels der Vorrichtung (1) zur Messung der flüchtigen Substanzen, wie in einem der Ansprüche 1 bis 12 festgelegt, umfassend die folgenden Schritte:
- Bereitstellung einer Sonde (80);
- Behandeln, beispielsweise durch Immersion, des Endes der Sonde (80) mit einer Probe einer zu untersuchenden Substanz, vorzugsweise eine flüssige Probe, wie beispielsweise Urin; und
- Einführen der Probe (80) durch die Analyseöffnung (71) in die Detektionsvorrichtung (1) wie in einem der Ansprüche 1 bis 12 festgelegt.

## Revendications

1. Dispositif pour la mesure de substances volatiles comprenant une boîte de confinement (20), ayant au moins un orifice d'entrée (30) pour l'introduction d'un flux de gaz, de préférence un air de qualité chromatographique, un ou plusieurs orifices de sortie (41, 42) dudit flux de gaz, et au moins un orifice d'analyse (71),
une pluralité de capteurs de détection (51, 52, 53, 54, 55, 56) disposés dans ladite boîte de confinement (20), et
au moins une sonde (80), pouvant être insérée dans ladite boîte de confinement (20) à travers ledit au moins un orifice d'analyse (71),
**caractérisé**
**en ce que** l'ouverture dudit orifice d'analyse (71) est plus petite que la somme des ouvertures desdits un ou plusieurs orifices de sortie (41, 42), de telle sorte que l'insertion ou l'extraction de ladite sonde (80) par ledit orifice d'analyse (71) ne change pas le flux dudit gaz qui atteint lesdits capteurs (51, 52, 53, 54, 55, 56),
**en ce que** ledit dispositif comprend un corps (73) disposé à l'intérieur de ladite boîte de confinement (20), ledit corps (73) étant pourvu d'un canal traversant (72) qui a une première extrémité communiquant avec ledit orifice d'analyse (71) et une deuxième extrémité communiquant avec l'intérieur de ladite boîte de confinement (20), et
**en ce que** ledit corps (73) présente radialement sur sa surface une pluralité de canaux, selon un nombre correspondant au nombre desdits capteurs (51, 52, 53, 54, 55, 56), lesdits canaux étant réalisés sur une paroi latérale du corps (73) et étant appropriés pour le partage du flux de gaz vers chaque capteur, de telle sorte que, lorsque ladite sonde (80) est préalablement traitée par un échantillon d'une substance à examiner et est insérée dans ladite boîte de confinement (20), ledit flux de gaz transporte uniformément les substances volatiles depuis ledit échantillon jusqu'à ladite pluralité de capteurs (51, 52, 53, 54, 55, 56).

2. Dispositif (1) selon la revendication 1, **caractérisé en ce que** le rapport entre ladite ouverture dudit orifice d'analyse (71) et la somme des ouvertures desdits un ou plusieurs orifices de sortie (41, 42) est inférieur et/ou égal à 0,01.

3. Dispositif (1) selon l'une quelconque des revendications précédentes, **caractérisé en ce que**
ladite boîte de confinement (20) a une base inférieure (21), dans laquelle est formée ledit orifice d'entrée (30), et une base supérieure (22), opposée à ladite base inférieure (21), dans laquelle sont formés lesdits un ou plusieurs orifices de sortie (41, 42) et ledit orifice d'analyse (71) pour l'introduction de ladite sonde (80), le nombre desdits orifices de sortie (41, 42) étant égal au nombre desdits un ou plusieurs capteurs (51, 52, 53, 54, 55, 56).

4. Dispositif (1) selon l'une quelconque des revendications précédentes, **caractérisé en ce que** ladite boîte de confinement (20) est un corps amovible.

5. Dispositif (1) selon l'une quelconque des revendications précédentes, **caractérisé en ce que** lesdits un ou plusieurs capteurs (51, 52, 53, 54, 55, 56) sont installés sur la surface latérale interne de ladite boîte de confinement (20).

6. Dispositif (1) selon l'une quelconque des revendications précédentes, **caractérisé en ce que** ladite boîte de confinement (20) a une forme cylindrique et ledit corps a une forme essentiellement sphéroïdale.

7. Dispositif (1) selon l'une quelconque des revendications précédentes, **caractérisé en ce que** ledit corps (73) a l'une des particularités suivantes :
- il est constitué d'un matériau isolant, de telle sorte que ledit échantillon d'une substance à examiner ne s'évapore pas lors de l'insertion de ladite sonde (80) à travers ledit canal (72) ; ou
- il est constitué de telle sorte que ledit canal traversant (72) ait une première partie (73') constituée d'un matériau isolant, de telle sorte que ledit échantillon d'une substance à examiner ne s'évapore pas lors de l'insertion de ladite sonde (80) dans la partie du canal débouchant (72) de ladite première partie (73'), et une deuxième partie (73") constituée d'un matériau conducteur, de telle sorte que, lors de l'insertion de ladite sonde (80) à travers la partie du canal débouchant (72) de ladite deuxième partie (73"), ledit échantillon d'une substance à examiner soit modifié à ladite température interne préalablement définie de ladite boîte de confinement (20).

8. Dispositif (1) selon l'une quelconque des revendications précédentes, **caractérisé en ce qu'**il comprend des moyens de répartition de flux (61, 62) disposés en correspondance dudit au moins un orifice d'entrée (30), lesdits moyens de répartition de flux (61, 62) étant appropriés pour répartir ledit flux de gaz à travers lesdits un ou plusieurs capteurs (51, 52, 53, 54, 55, 56),
lesdits moyens de répartition de flux comprenant un séparateur poreux (61), de préférence constitué de verre, disposé de façon à délimiter une chambre de diffusion (D) dudit gaz en correspondance dudit orifice d'entrée (30) par rapport une chambre de mesure et de vaporisation (M), dans laquelle les substances volatiles dudit échantillon s'évaporent et sont transportées par ledit flux de gaz, par lesdits canaux dudit corps central, l'échantillon étant envoyé vers lesdits capteurs (51, 52, 53, 54, 55, 56).

9. Dispositif (1) selon la revendication 8, **caractérisé en ce qu'**il comprend des moyens de régulation de la température interne de ladite boîte de confinement (20), lesdits moyens de régulation comprenant de préférence une première thermistance installée dans ladite chambre de diffusion (D) et une deuxième thermistance installée dans ladite chambre de mesure et de vaporisation (M).

10. Dispositif (1) selon l'une quelconque des revendications précédentes, **caractérisé en ce que** ladite substance à examiner est un liquide, tant sous forme liquide que sous forme de vapeur.

11. Dispositif (1) selon l'une quelconque des revendications précédentes, **caractérisé en ce que** ladite substance à examiner est un solide.

12. Dispositif (1) selon l'une quelconque des revendications précédentes, **caractérisé en ce qu'**il est pourvu d'un système de batterie Peltier pour le refroidissement de ladite au moins une sonde (80) avant l'échantillonnage d'une substance volatile et l'insertion de celle-ci à travers ledit au moins un orifice d'analyse (71).

13. Appareil de détection et de mesure **caractérisé en ce qu'**il comprend :
au moins un dispositif de détection et de mesure (1) tel que défini dans l'une quelconque des revendications 1 à 12 ;
des moyens d'amenée dudit flux de gaz, comprenant une bouteille à gaz, connectés audit orifice d'entrée (30) par une conduite (31) ;
des moyens d'ajustement de la température dudit gaz sortant desdits moyens d'amenée dudit flux de gaz ;
au moins un dispositif de réduction de la pression dudit gaz ;
au moins un débitmètre pour l'ajustement dudit gaz ; et
une unité de commande pourvue d'un écran à cristaux liquide et d'éléments d'interaction, ladite unité de commande étant connectée auxdits moyens d'amenée dudit flux de gaz, auxdits capteurs (51, 52, 53, 54, 55, 56), auxdites première et deuxième thermistances, auxdits moyens d'ajustement de la température, audit au moins un dispositif de réduction de la pression et audit débitmètre, ladite unité de commande étant appropriée pour détecter la température dudit gaz, pour la comparer à la température de ladite chambre de diffusion (D) et de ladite chambre de mesure et de vaporisation (M), de façon à ajuster lesdites températures par lesdits moyens d'ajustement de la température et lesdites thermistances, pour détecter les mesures desdits capteurs (51-56), et pour présenter les résultats sur ledit écran.

14. Procédé de détection de substances volatiles au moyen dudit dispositif (1) pour la mesure de substances volatiles tel que défini dans l'une quelconque des revendications 1 à 12, comprenant les étapes suivantes :
- fourniture d'une sonde (80) ;
- traitement, par exemple par immersion, de l'extrémité de ladite sonde (80) dans un échantillon d'une substance à examiner, de préférence un échantillon liquide, telle que de l'urine ; et
- introduction de ladite sonde (80) dans ledit dispositif de détection (1) tel que défini dans l'une quelconque des revendications précédentes 1 à 12 à travers ladite ouverture d'analyse (71).
